# EUROPEAN PATENT APPLICATION

(11) **EP 2 727 592 A1**
(43) Date of publication of application: **07.05.2014**
(21) Application number: 13188409.0
(22) Date of filing: 14.10.2013
(51) Int. Cl.: A61K 31/4545, A61K 31/167

(54) **A combination of desloratadine and paracetamol**

(30) Priority: 05.11.2012 TR 201212712
(71) Applicant: Kücükgüzel, Sükriye Güniz, Istanbul (TR)
(72) Inventor: Küçükgüzel, Sükriye Güniz, Istanbul (TR); Bas, Kaan, Istanbul (TR)
(74) Representative: Mutlu, Aydin

(57) **Abstract**

The present invention is related to a combination formulation formed of two active materials and an auxiliary agent being antihistaminic Desloratadine and/or pharmaceutically acceptable salts thereof and Paracetamol which is a painkiller and a fever reducer.

## Description

### Technical Field

The present invention is related to a combination formulation formed of two active materials and an auxiliary agent being antihistaminic Desloratadine and/or pharmaceutically acceptable salts thereof and Paracetamol which is a painkiller and a fever reducer. As the target group is children in this study alcohol has not been used during the formulation stage.

### Prior Art (Known state of the art)

Paracetamol (4-hydroxyacetanilide) (PAR) (Formula I), is in the group of fever reducer, pain killer non narcotic and non steroid analgesics. Paracetamol prevents toothaches, headaches and flu symptoms and fever effects.

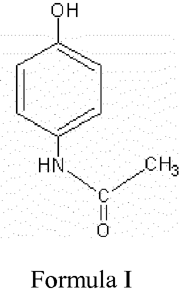

Following taking PAR, at a customary dose orally, nearly all of it is absorbed through the gastrointestinal channel and within 30 minutes, it will start showing analgesic effects and in 90 minutes it will reach the highest amount of plasma concentration. The analgesic effect will last for 3 - 4 hours. Oral bioavailability is 63 - 89% and the rate of exposure to the first effect of passing through the liver is low. Elimination half life is 3.3 hours.

In pains connected with dysmenorrhoea, the analgesic effect of 1000 mg PAR used on its own is lower than 400 mg naproxen. In the multi centred, randomized, double blind, parallel group, comparison study, a difference in terms of analgesic efficiency between patient groups using 4000 mg PAR per day for osteoarthritis and other patients using 750 mg naproxen has not been found and gastrointestinal bleeding and anaemia side effects connected to the usage of naproxen has been stated. In another study, a difference between orally used PAR in terms of postoperative analgesic efficiency, diclofenac and PAR-diclofenac combination has not been observed. PAR, PRO (Propiphenazone) and KAF (Caffeine) have potential synergic effect. PAR, PRO and KAF used in combination is more effective then PAR, Ibuprofen and aspirin used on their own.

PAR, is much cheaper than many analgesic medicine and it can be safely used during pregnancy (safety category B), and it is an analgesic which does not have teratogenic effect and it is used more than aspirin and ibuprofen. The analgesic effect mechanism of PAR which is one of the oldest and safest painkillers is still not clearly known. Although it is thought to be within non steroidal anti-inflammatory medicines when classified, PAR is different in terms of its side effects when compared with other medicines within said classification. In previous years it has been put forward that PAR showed effect by inhibiting the peripheral prostaglandin synthesis. However in the studies carried out in the recent years it has been stated that PAR has a weak inhibition effect on peripheric prostaglandin and that it provides analgesic effect by inhibiting some forms of central cyclooxygenase enzymes. Lee et al., have put forward that PAR has a peripheric effect and it inhibits a sub class of COX-2. The type named COX-3 of Cyclooxygenase enzyme plays a role in the synthesis of prostaglandins in the brain. According to the studies carried out in the recent years it has been shown that PAR does not inhibit the enzymes of COX-1 and COX-2 but shows analgesic and antipyretic effect with COX-3 inhibition. This explains why the PAR has anti-inflammatory effect and does not have gastrointestinal side effects. The reason for this is that COX-1 inhibition has gastrointestinal side effects and COX-2 inhibition shows anti-inflammatory effects.

Organum vasculosum laminae terminalis (OVLT) is an organ which is located on the front wall of the third cerebral ventricle and is the centre where the body temperature is regulated. According to the tests carried out on rabbits staphylococcus enterotoxin-A increases the glutamate concentration in this organ and the body temperature rises. PAR inhibits the glutamate release in OVLT and shows antipyretic effect.

As its anti-inflammatory effect is low, PAR is not used in indications which show this type of efficiency. However, it can be used together with anti-inflammatory medicines to increase their effect. As a result of using PAR together with non steroidal anti-inflammatory medicine besides PRO, there is no proof that extra analgesic effect will be formed or not. Its antithrombotic efficiency is low; thus it does not change the haemorrhage time. PAR does not have a significant effect on the cardiovascular system, acid base balance and respiration. It does not cause irritation or bleeding in the stomach. It does not affect Protrombin synthesis. It does not bind to plasma proteins much. PAR is given between 500 - 1000mg dose to adults and this dose will be repeated if necessary every 4 - 6 hours. The highest daily dose is accepted to be 4 g. It has been stated to be 3 g, in some sources and 2,6 in other sources. The dose must be decreased for people who have renal failure and in alcoholics. It is not advised to be used in such doses for more than 10 days. As it has a lower hepatoxicity potential in children, dose per kg is higher and 10 mg kg¹ can be administered in one go. 30 mg kg⁻¹ dose PAR in one go can be given to children between the age of 6 - 12. A onetime dose for babies younger than 1 year is 60 - 120mg and 120 - 250 mg for children between the age of 1 - 5. For a higher amount of bioavailability, it is advised that orally used PAR is taken before meals. PAR's side effects are rarely seen when taken in customary doses, however allergic reactions have been noted (skin rash and urticaria). Nearly all of the PAR is metabolized in the liver and 5 % is eliminated without transformation, 90 % turns into glucuronide and sulphate conjugate and 5 % turns into N-acetyl-p-benzoquinonimine (NAPQI) with oxidation. NAPQI, is a reactive material, it is hepatoxic and turns into glutathione conjugate and following this it will turn into harmless cystine and mercapturate and is then eliminated. However when the PAR dose increases NAPQI formation increases and when the glutathione stock in the liver is consumed NAPQI is bound to mithocondria and core proteins of liver cells via covalent connections. As a result liver necrosis occurs. PAR when taken in adults over 10 g, will lead to nausea, vomiting and stomach ache within 24 hours and can then turn into jaundice, liver failure, hepatic encephalopathy and acute liver necrosis prognostic with renal failure. If over 20g of PAR is taken in one go, the fatality risk shall increase. If taken 5 - 8 g dose daily for a few weeks it could cause liver necrosis and death in connection with this. Children are more resistant to the hepatoxic effect of high doses of PAR. PAR's paediatric toxic dose has been noted to be 200 mg/kg. In PAR toxicity SH- donor is present and N-acethylsistein which increases the amount of glutathione is administered intravenously.

Desloratadine (8-Chloro-6,11-dihydro-11-(4-piperidinylidene)-5H-benzo[5,6]cyclohepta[1,2-*b*]pyridine) (Formula II) is an active material that is used in treating allergic indications and which is within the group of H1 receptor antagonists which is a non-sedative antihistaminic and prevents the effects of edema, redness of the skin, fever, itching, watery eyes, and runny nose which can be caused by histamine which is a natural chemical in the body. Desloratadine belongs to the group of antihistaminic medicine and it is a second generation antihistaminic which does not cause sedation.

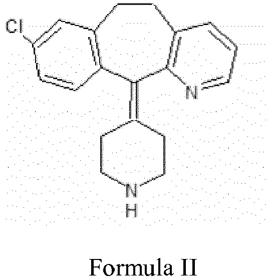

The advantages of Desloratadine can be summarized as follows:
- Its bioavailability will not be effected by food
- It is effective for 24 hours
- It does not cause sedation
- It does not cause psychomotor and cognitive function anomalies
- The psychomotor activities of alcohol does not increase its disruptive effect
- It does not form an anticholinergic effect
- It will not cause weight gain

Various polymorphic structures of Desloratadine in literature have been mentioned. In the study where a single crystal X-ray diffraction analysis is carried out for a Form I structure of Desloratadine by Prashant M. Bhatt and Gautam R. Desiraju the unit cell stacking and crystal structure has been illuminated. It has been determined that there is a hydrogen connection only in one of the structure of pyridine N-H and other molecule benzene ring hydrogen. It has been noted that this situation is the cause for the molecule to be stacked like a zig zag shape. In this study, it is interestingly noted that in the Form I crystal structure the hydrogen bond is determined not to be between the piperidine ring (N-H....N) of two separate molecules. They have also indicated that this situation is in harmony with the calculations carried out with the polymorph predictor program in a computer medium.

In a study carried out in the year 2010, a series of desloratadine derived stereo have been selectively synthesized and H₁ antihistaminic activities have been determined. This has shown that the derivatives are substantially connected to the substituent in the lacton ring of H₁-antihistaminic activities. The derivatives in the study carried out have been shown below.

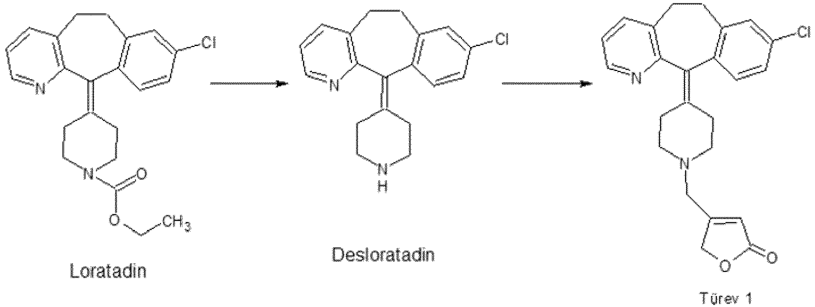

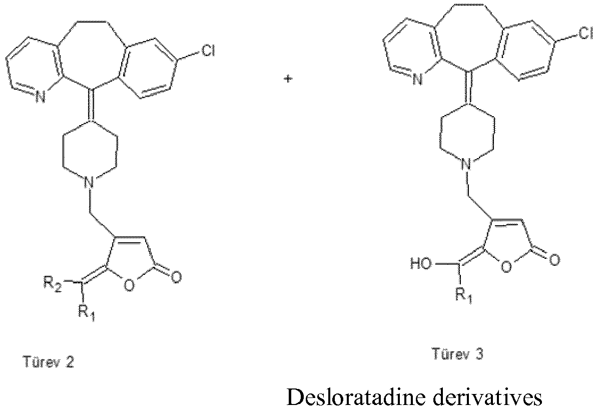

In the patent application numbered WO 2005/084674 it is aimed to determine the structure of desloratine during the solid phase, desloratadine is stirred for 5 - 10 minutes at 25 - 35 °C and is dissolved in methanol, the dissolved solution which is distilled is dried under nitrogen pressure with a spray dryer which has an air inlet temperature of approximately 105°C, and an air discharge temperature of approximately 70° C. By this means amorphous desloratadine has been obtained. In the patent numbered US 6,506,767 B1 a 24 hour time range has been taken as basis and it has been determined that Desloratadine is an antihistamine which does not have a sedative effect, that its side effect is less than loratadine and that it is 2,5 to 4 times more effective than loratadine. Desloratadine has been obtained by using different solvents together with sodium hydroxide and potassium hydroxide from loratadine according to the patent application numbered WO2004/029039 A1. The efficiency of desloratadine obtained with these synthesis and reaction times have been compared.

It has been noted for a long time in various studies that desloratadine which is the active ingredient of the medicine can be found in two different forms commercially. These have been named as Form I and Form II. In the patent application numbered WO 99/01450 it has been determined that when desloratadine is crystallized with halogenous hydrocarbon solvents (for example dichloromethane) or methyl isobutyl methyl ketone solvents, crystals which have a high rate of form I can be obtained. Form II on the other hand can be obtained at a higher amount by either heating desloratadine for half an hour at - 160 °C temperature and melting it and the slowly cooling it back to have crystallization or crystallizing with ethyl acetate in a controlled manner. In the patent numbered US 6,506,767 B1 regarding the determination of the stability of the Form I structure of desloratadine, respectively calculation of amounts in humid medium containing 25 °C , 30 °C and 40 °C'de % 60 Rh and % 60 - % 75 Rh humidity, total impurity, X ray diffraction, FT-IR spectrophotometer, pH analysis has been carried out and changes lower than 1% have been observed. When hexane or heptanes mixtures were used in different rates as crystallization solvents, different rates of Form I: Form II mixtures have been obtained. Desloratadine has been heated under re-cooler inside ethyl asetate and drained with active coal and following the washing and drying carried out with hexane, a Form I: Form II mixture with a high rate of Form II has been obtained.

Desloratadine which has a structure of Form I is a much more steady form when compared with the desloratadine structure within form II. When Form II rate increases in the mixtures, colouring in the product can be seen in time. This colouring is thought to be due to the formation of impurity and increase in impurities. In this case Form while Form I can be stored in room temperature, Form II needs to be stored in lower temperatures (for example 2-8 °C) with its lid closed and by being protected from light.

### Metabolism:

Desloratadine is actually an active metabolite of loratadine. In vitro dependent efficiency power is 10 - 20 times more in comparison to loratadine. Loratadine and desloratadine are antihistaminics that do not cause sedation: however desloratadine will not lengthen the QT distance even if given 4 - 9 times more than the doses suggested for adults. Desloratadine's main metabolic route is hydro circulation. The medicine is hydroxilated in order to form 3-hydroxy desloratadine metabolites. The glucuronide conjugate formed by the glucuronation of this metabolite, will be eliminated by means of urine and bile. The enzyme that is responsible for Desloratadine metabolism has still not been determined. For this reason interaction with other medicines must be taken into consideration. The studies carried out have shown that CYP3A4 and CYP2D6 enzymes were not effective on desloratadine metabolism. Desloratadine inhibits CYP3A4 or CYP2D6. It is not a P-glycoprotein substrate or inhibitor. In the studies carried out, an effect on the disposition of desloratadine to food and grapefruit has not been found.

### Description of the invention

The present invention Desparas is related to a combination formulation made of pharmaceutically acceptable one or more excipients and two active ingredients one being antihistaminic desloratadine and/or its pharmaceutically acceptable salts and pain killer and fever reducer Paracetamol. In this study, as the target group is children during the formulation stage, any kind of alcohol has not been used.

The excipients used in the study are propylene glycol, sorbitol (70 %), Lycasin, sodium, saccharin, sodium citrate, citric acid, anhydride, strawberry flavour, Panceu 4R as colorant.

### Analytic methods used

### Chromatographic conditions:

| | |
|---|---|
| **Column** | : Luna 5 µ Phenyl-Hexyl 250 x 4.6 mm |
| **Flow rate** | : 2 ml/min. |
| **Detector** | : U.V. |
| **Wave length** | : 254 nm |
| **Injection volume** | **:** 15 µl |
| **Mobile phase** | : Tampon solution: Acetonitrile (70 : 30 , h/h) |
| **Temperature** | : 25 °C |
| **Retention period** | **: ~**3.5 minutes |

### Preparation of the mobile phase:

KH₂PO₄ (6.8 g) has been weighed, has been dissolved in 1000 ml distilled water, and will be adjusted to 3 in pH with H₃PO₄ concentration. It is sieved through a 0.45 µm nylon filter. 700 ml will be taken from this solution and it will be mixed with 300 ml acetonitrile.

### Dilution solvent: Is mixed with 500 ml acetonitrile and 500 ml distilled water

### Standard Preparation:

As study standards, Desloratadine (5.0 mg) and Paracetamol (250.0 mg) is weighed into 100 ml volumetric flask, its volume will be completed to 100.0ml with acetonitrile. It is kept for 15 minutes in an ultrasonic bath. It is cooled. 10 ml will be extracted with a straw and is kept in a 100 ml volumetric flask and it is topped up to 100 ml with dilution solution. It is mixed for 10 minutes in a magnetic mixer. And it is sieved out of a 0.45 µm PET filter.

### Preparation of a sampler:

10 ml sample is taken into a 100 ml volumetric flask by means of a straw and it is topped up to 100 ml with acetonitrile. It is kept in an ultrasonic bath for 15 minutes. It is cooled. 10 ml will be extracted with a straw from this solution and it is topped with dilution solvent in a 100 ml volumetric flask. It is mixed in a magnetic mixer for 10 minutes. It is sieved through a 0.45 µm PET filter.

### EXAMPLES:

### EXAMPLE 1:

125 g Paracetamol, 350 g Propylene glycol has been dissolved at 70 C° in a beaker and a very light pinkish clear solution has been established. 0.5 g Desloratadine has been added on this and it is mixed. Later on the temperature is decreased to 40 C° in an ice bath, 225 g 70 % sorbitol, 200 g glycerine is added and mixed. Following this 0.4 g sodium saccharin is dissolved in 10 ml distilled water, on the other hand 0.02 g sodium citrate is dissolved in 10ml distilled water and is added. 100 ml distilled water is added on top. And finally 0.64 g anhydrite citric acid is dissolved in 100 ml distilled water and pH has been adjusted to: 4.00. 0.04 g Ponceau 4R Lake and 1.7 ml milk vanilla essence has been added.

**RESULT:** After is left to rest in room temperature for a day, it has been observed that paracetamol has precipitated.

### EXAMPLE 2:

Due to the initial precipitation of the first product, it was thought that Desloratadine and Paracetamol went into reaction and this was why precipitation occurred and each one is dissolved alone, later it is mixed and it was noted that Paracetamol again precipitated during the mid way of the production and production was completed.

**RESULT:** It has been understood as a result of this production that Paracetamol precipitation was sourced out from Desloratadine. As an action paracetamol solution amount has been kept at a maximum and new production has been started.

### EXAMPLE 3:

The propylene glycol amount in this production is kept at a maximum (decreasing the amounts of water and glycerine) and a new production has been started. However after 70 C° was reached 70% sorbitol was added and following this when it was cooled down to room temperature, again it has been noted that Paracetamol would crystallize and precipitate.

**RESULT:** The result that has been reached according to these studies show that 125 mg/ml paracetamol used for the formulation was too much and this was why precipitation occurred and a change in the formulation was carried out.

### EXAMPLE 4:

Production has been carried out in the formulation having 0.5 mg/ml Desloratadine and 25 mg/ml Paracetamol. 175 g Propylene Glycol was placed into a 2 It beaker and following this 25 g Paracetamol was added and it was heated until 70 C° and dissolved and 0.5 g Desloratadine was added and dissolved in magnetic mixer. Following this the temperature is increased up to 80 C° 510 g Lycasin was added and heated up to 80 C° and 100 g 70 % Sorbitol was added and cooled to 40 C°. Following this 0.8 g sodium saccharine was dissolved in 15 ml distilled water, 0.15g sodium citrate was dissolved in 15ml distilled water, 0.71 anhydride citric acid was dissolved in 20 ml distilled water and then sodium saccharin was added and mixed and following this sodium citrate was also added and mixed and in order to achieve pH adjustment pH was adjusted to approximately 3.99 with anhydrite citric acid. As a colorant 0.03 g Panceu 4R lake and 2.7 ml strawberry flavour was added and mixed. After this 260 ml distilled water was topped up. As a result 2 It pilot production was obtained.

**RESULT:** After monitoring precipitation was not observed and it has been determined to be the final formula. In this study a combined product study was carried out between desloratadine which did not have a medicinal reaction and/or its pharmaceutically acceptable salts and paracetamol (DESPARAS) and an analytic method was developed. During the Desperas trial studies in the first trial a significant amount of precipitation following the 1 hour waiting period after the pilot production was completed was observed. It has been believed through this finding that Paracetamol and Desloratadine interacted. In order to resolve this disadvantage it has been decided to dissolve these two active ingredients in separate solvents and then to mix them. Precipitation was observed again before even completing the production and the production was left incomplete. A new trial was then carried out by taking this into consideration. The solvent amount used to dissolve paracetamol was maximized and the production was tried again. As a result precipitation was again observed. It has been realized that the mistake in the formula was due to the paracetamol amount as the paracetamol syrup formulations that were being marketed contained at most 1ml/32 mg paracetamol. In accordance with the result obtained here, Desparas syrup formulations were changed and a new syrup formulation having 0.5 mg Desloratadine per millimetre and 25 mg Paracetamol was tried. In the fourth pilot production that was carried out precipitation was not observed and the 21t pilot study was bottled and were stored in 25 °C, 30 °C and 40°C'stability cabins. The stability analytic results have been given in Table 1. Moreover the three bottles were kept in room temperature and one bottle was subjected to an opening and closing test (the bottle was opened and closed three times a day); following this test any form of crystallization near the edges of the bottle top was not observed and as a result at the end of the tenth day crystallization was not observed. The other bottle was just observed. Following a month of observation a change in the appearance of desparas was not seen. Taste and scent test was carried out for the other bottle, and following a month of tracking a negative result was not determined.

As a result: it has been decided that the formula for Desparas was suitable comprising 0.5 mg Desloratadin-25 mg Paracetamol/ml.

**Table 1 Desparas Analytic Findings**

| **Tests** | **Limits** | **Start** | **3.Months (25 °C)** | **3.Months (30 °C)** | **3.Months (40 °C)** |
|---|---|---|---|---|---|
| Appearance | Red coloured clear, liquid with strawberry scent. | Suitable | Suitable | Suitable | Suitable |
| pH | Approx. 3.5 - 4.5 | 3.99 | 3.97 | 3.97 | 3.96 |
| Paracetamol M.T. | % 95 - % 105 | % 98.3 | % 98.1 | % 97.9 | % 97.8 |
| Desloratadine M.T. | % 95 - % 105 | % 98.9 | % 98,7 | % 98.6 | % 98.3 |

## Claims

1. An oral pharmaceutical composition **characterized in that**; it comprises paracetamol and/or pharmaceutically acceptable salts thereof and/or derivatives and desloratadine and/or pharmaceutically acceptable salts thereof and/or derivatives and one or more pharmaceutically acceptable auxiliary agent.

2. A syrup composition comprising paracetamol and desloratadine for oral use.

3. An oral pharmaceutical composition **characterized in that** the amount of desloratadine inside a millilitre is 0.5 mg and the amount of paracetamol is 25 mg.

4. A composition according to claims 1 and 3, **characterized in that** the auxiliary agents within the formulation are propylene glycol, sorbitol, Lycasin, sodium saccharin, sodium citrate, citric acid anhydrite, strawberry flavour and Panceu 4R Lake as a colorant.

5. An oral pharmaceutical composition according to any of the preceding claims **characterized in that** the pH of the composition is approximately 3.5 - 4.5.

6. An oral pharmaceutical composition according to any of the preceding claims **characterized in that** said composition does not comprise alcohol.
